# EUROPEAN PATENT SPECIFICATION

(11) **EP 1 623 030 B1**
(45) Date of publication and mention of the grant of the patent: **29.11.2006**
(21) Application number: 04731658.3
(22) Date of filing: 07.05.2004
(51) Int. Cl.: C12N 9/90, G01N 33/50

(54) **TOPOISOMERASE HYBRIDS AND METHODS OF USE**
HYBRID TOPOISOMERASEN UND IHRE VERWENDUNG
TOPOISOMERASES HYBRIDES ET LEURS PROCEDES D'UTILISATION

(30) Priority: 09.05.2003 US 469457 P
(43) Date of publication of application: 08.02.2006
(73) Proprietor: AstraZeneca AB, 151 85 Södertälje (SE)
(72) Inventor: EAKIN, Ann, Waltham, MA 02451 (US); EHMANN, David, Waltham, MA 02451 (US); GAO, Ning, Waltham, MA 02451 (US); KARANTZENI, Irene, Waltham, MA 02451 (US); WALKUP, Grant, Waltham, MA 02451 (US)
(86) International application number: PCT/GB2004/001980
(87) International publication number: WO 2004/099397

(56) References cited:
- US-A- 6 001 631
- SIMON HANNELORE ET AL: "Biochemical complementation studies in vitro of gyrase subunits from different species" FEBS LETTERS, vol. 373, no. 1, 1995, pages 88-92, XP002299389 ISSN: 0014-5793 cited in the application
- BLANCHE FRANCIS ET AL: "Differential behaviors of Staphylococcus aureus and Escherichia coli type II DNA topoisomerases" ANTIMICROBIAL AGENTS AND CHEMOTHERAPY, vol. 40, no. 12, 1996, pages 2714-2720, XP002299390 ISSN: 0066-4804 cited in the application
- KATO JUN-ICHI ET AL: "Purification and characterization of DNA topoisomerase IV in Escherichia coli" JOURNAL OF BIOLOGICAL CHEMISTRY, vol. 267, no. 36, 1992, pages 25676-25684, XP002299391 ISSN: 0021-9258 cited in the application

## Description

### FIELD OF THE INVENTION

The present invention relates to hybrid topoisomerases, methods for assaying topoisomerase activity, and methods for identifying compounds that modulate topoisomerase activity

### BACKGROUND

The continuing development of resistance to existing antibacterial agents is a serious medical problem that is worsening. In order to maintain effective treatments for bacterial ailments it is necessary to develop new drugs that attack bacteria through interactions with new target proteins or with new classes of compounds that exploit validated chemotherapeutic targets (Sefton, 2002, Drugs, 62:557-566).

Prokaryotic (bacterial) type II topoisomerases are enzymes that modify the topological state of double stranded closed circular DNA at the expense of adenosine triphosphate (ATP) hydrolysis. DNA gyrase (gyrase) and topoisomerase IV (topo IV) are both bacterial type II topoisomerases. These enzymes are tetrameric, composed of two distinct monomers each in duplicate. Gyrase is composed of the proteins GyrA and GyrB; Topo IV is composed of the proteins ParC and ParE. For the organism *Staphylococcus aureus* (*S. aureus*), the ParC protein is known interchangeably as either ParC or GrIA and the ParE protein is known interchangeably as either ParE or GrIB. In addition, GyrA and ParC are homologous as are GyrB and ParE. It is well known that GyrA and ParC interact with DNA whereas GyrB and ParE contain the ATP hydrolysis function of the enzyme. Thus, GyrA and ParC are known as DNA binding (and cleaving) subunits of type II topoisomerases and GyrB and ParE are known as ATP hydrolyzing subunits of type II topoisomerases. Several reviews of these enzymes have been published (see for example: Champoux, 2001, Annu. Rev. Biochem., 70:369-413; Reece & Maxwell, 1991, Crit. Rev. Biochem. Mol. Biol., 26:335-375).

The quinolone antibiotics are medically and commercially relevant drugs that inhibit gyrase and/or topo IV. These drugs are known to specifically bind the GyrA or ParC subunit of the enzyme, remote from the ATP binding site of the enzyme. Agents that modulate the ATP-hydrolyzing function of type II topoisomerases are also known and include the cyclothialidine family as well as the coumarins (novobiocin, chlorobiocin, coumermycin A₁). These natural products are potent inhibitors of the ATPase activity of gyrase and have been shown to bind within and adjacent to the ATP binding pocket of the enzyme (Lewis *et al.,* 1996, EMBO J., 15:1412-1420). In addition, novobiocin has been applied clinically as an antibacterial agent, although with limited scope. Nonideal physical and pharmacological properties of this agent have tempered its use as a drug but validated the concept of inhibiting the ATPase of gyrase for the treatment of bacterial infections. As a consequence, there continues to be interest in the pharmaceutical industry to develop drug candidates that inhibit gyrase ATPase activity (WO 99/49077; WO 99/46595; US 5,998,152; US 6,197,527; US 6,608,087; US 6,632,809; Annedi & Kotra, 2001, Curr. Opin. Investig. Drugs, 2:752-754; Lafitte *et al.,* 2002, Biochemistry, 41:7217-7223; Boehm *et al.,* 2000, J. Med. Chem., 43:2664-2674).

An extensive body of knowledge exists in the literature concerning the ATP hydrolyzing function of gyrase, GyrB, and fragments thereof for the *Escherichia coli* (*E. coli*) enzyme. By comparison, much less biochemical characterization of the ATP hydrolyzing subunits of other type II topoisomerases has been reported. While full length and N-terminal protein fragments of the *E. coli* GyrB protein show high activity of ATP hydrolysis, isolated *S. aureus* GyrB and *S. aureus* ParE do not.

For the development of new antibacterials, agents that are active against a broad range of bacterial species are commonly desired. Accordingly, having an assay for modulators of a Gram positive type II topoisomerase ATPase (for example, *S. aureus*) as well as a Gram negative (for example, *E. coli*) is particularly valuable. Studies have been reported where gyrase A and B subunits from different species of bacteria are mixed (Simon *et al.,* 1995, FEBS Lett., 373:88-92; Brown *et al.,* 1979, Proc. Natl. Acad. Sci. USA, 76:6110-6119; Orr & Staudenbauer, 1982, J. Bacteriol., 151:524-527). Additionally, attempts to mix gyrase and topoIV subunits from *E. coli* have been reported (Kato *et al.,* 1992, J. Biol. Chem., 267:25676-25684). There is a need to identify new compounds that modulate prokaryotic topoisomerases for use in treating bacterial infections.

### SUMMARY

The present invention provides hybrid topoisomerases comprising a DNA binding subunit of a type II topoisomerase from a Gram-negative prokaryote and an ATP-hydrolyzing subunit of a type II topoisomerase from *S. aureus.* In some embodiments, the Gram-negative prokaryote is selected from Enterobacteriaceae, Pseudomonadaceae, Bacteroides species, Haemophilus species, Helicobacter species, Neisseria species, *Campylobacter jejuni,* Legionella species, and *Moraxella catarrhalis.* In some embodiments, the Gram-negative prokaryote is *E. coli.* In some embodiments, the type II topoisomerase DNA binding subunit is GyrA from *E. coli.* In some embodiments, the ATP-hydrolyzing subunit is *S. aureus* GyrB or ParE. In further embodiments, the hybrid topoisomerase comprises *E. coli* GyrA and *S. aureus* GyrB. In further embodiments, the hybrid topoisomerase comprises *E. coli* GyrA and *S. aureus* ParE.

The present invention also provides methods for assaying topoisomerase activity comprising providing a hybrid topoisomerase comprising a DNA binding subunit of a type II topoisomerase from a Gram-negative prokaryote and an ATP-hydrolyzing subunit of a type II topoisomerase from *S. aureus,* and determining topoisomerase activity. In some embodiments, the methods further comprise contacting the hybrid topoisomerase with DNA. In some embodiments, topoisomerase activity is determined by detecting a change in topology of the DNA. In some embodiments, the change in DNA topology is determined by detecting DNA relaxation, DNA supercoiling, or DNA decatenation. In some embodiments, topoisomerase activity is determined by detecting ATPase activity. In some embodiments, ATPase activity is detected by measuring inorganic orthophosphate or adenosine diphosphate.

The present invention also provides methods for identifying compounds that modulate topoisomerase activity comprising: a) providing a hybrid topoisomerase comprising a DNA binding subunit from a prokaryotic type II topoisomerase and an ATP-hydrolyzing subunit from a prokaryotic type II topoisomerase; b) contacting the hybrid topoisomerase with a test compound; and c) determining topoisomerase activity, wherein a change in topoisomerase activity in the presence of said compound as compared with topoisomerase activity in the absence of said compound indicates that said compound modulates topoisomerase activity. Some embodiments further comprise contacting the hybrid topoisomerase with DNA and a test compound. In some embodiments, topoisomerase activity is determined by detecting a change in topology of the DNA. In some embodiments, the change in DNA topology is determined by detecting DNA relaxation, DNA supercoiling, or DNA decatenation. In some embodiments, topoisomerase activity is determined by detecting ATPase activity. In some embodiments, ATPase activity is detected by measuring inorganic orthophosphate or adenosine diphosphate. In some embodiments, the assay further comprises determining if the compound has antibacterial activity. In some embodiments the DNA binding subunit is *E. coli* GyrA and the ATP-hydrolyzing subunit is *S. aureus* GyrB. In some embodiments, the DNA binding subunit is *E. coli* GyrA and the ATP-hydrolyzing subunit is *S. aureus* ParE.

### BRIEF DESCRIPTION OF THE DRAWINGS

Figure 1 shows a typical IC₅₀ measurement for novobiocin, a natural product inhibitor gyrase, under typical assay conditions.
Figure 2 shows the effect of equimolar *E. coli* GyrA on the activity of *S. aureus* GyrB relative to other treatments.
Figure 3 shows the effect of varying quantities of *E. coli* GyrA as an activator of *S. aureus* GyrB in the presence or absence of DNA.
Figure 4 shows the ATP-dependent supercoiling of plasmid DNA for the hybrid *E. coli* GyrA and *S. aureus* GyrB, with the ATP-dependent supercoiling effect of *E. coli* gyrase for comparison.
Figure 5 shows the effect of equimolar *E. coli* GyrA on the activity of *S. aureus* ParE relative to other treatments.

### DETAILED DESCRIPTION

The present invention provides, in part, hybrid toposiomerases having enhanced ATPase activity and that are useful for screening for compounds that modulate (activate, enhance or inhibit) the activity of topoisomerases. The methods of the present invention are amenable to high- throughput screening formats.

We have found that combining DNA binding subunits with ATP hydrolyzing subunits from different type II topoisomerses enhances the ATPase activity. For example, we have found that combining *E. coli* GyrA (the DNA binding subunit from *E. coli*) with *S. aureus* GyrB (the ATP hydrolyzing subunit from *S. aureus*) enhances the ATPase activity of the *S. aureus* GyrB protein. Additionally, we have found that combining *E. coli* GyrA with *S. aureus* ParE (the ATPase subunit of topoisomerase IV) greatly enhances the ATPase activity of the *S. aureus* ParE protein. For example, the enhancement of ATPase activity makes possible sensitive assays of the ATPase activity of *S. aureus* topoisomerases. Further, the assays of the invention can be used for screening for changes in DNA topology. For example, the present invention provides screening assays to identify compounds that function to modulate the activity of *S. aureus* type II topoisomerases, by using, for example, *E. coli* GyrA to enhance the activity of *S. aureus* ATP hydrolyzing subunits.

As used herein, the term "hybrid topoisomerase" refers to a type II topoisomerase enzyme comprizing a DNA binding subunit and an ATP hydrolyzing subunit that are not complexed together in nature. For example, "hybrid topoisomerase" includes a DNA binding subunit and an ATP hydrolyzing subunit brought together from two different prokaryotic species, such as a hybrid comprising *E. coli* GyrA and *S. aureus* GyrB or a hybrid comprising *E. coli* GyrA *S. aureus* ParE. "Hybrid topoisomerase" also includes a DNA binding subunit and an ATP hydrolyzing subunit brought together from two different type II topoisomerases of the same prokaryotic species, for example, a hybrid comprising *S. pneumonia* GyrA (gyrase) and *S. pneumonia* ParE (topoIV).

For example, hybrid topoisomerases formed from *E. coli* GyrA and *S. aureus* GyrB or *S. aureus* ParE provide an active ATPase that can conveniently be assayed by standard methods (for example, malachite green) at single-nanomolar levels of enzyme. Completely formatted assays using hybrid topoisomerases permit the identification of compounds that modulate the ATPase activity of the hybrid topoisomerases. Such modulation of topoisomerase activity can also be monitored by assaying DNA topology. For example, the hybrid gyrase comprising *E. coli* GyrA and *S. aureus* GyrB displays ATP-dependent DNA supercoiling activity, which is the complete reaction of the native gyrase enzyme. Therefore, standard assays that measure ATP-dependent DNA supercoiling activity can be used to identify compounds that modulate the enzyme; and more particularly to identify compounds that inhibit the enzyme.

Hybrid topoisomerases of the present invention can be used to assay and monitor topoisomerase activity. Hybrid topoisomerases of the present invention can be used in assays to identify compounds that modulate topoisomerase activity.

In one aspect, the present invention provides hybrid topoisomerases comprising a DNA binding subunit of a type II topoisomerase from one source (species or topoisomerase) and an ATP-hydrolyzing subunit of a type II topoisomerase from another source (species or topoisomerase).

Type II topoisomerase subunits from any prokaryote can be used in the hybrid topoisomerases and in the assays of the invention, including but not limited to the family Enterobacteriaceae (such as *E. coli, Klebsiella pneumoniae,* and *Salmonella typhimurium*)*,* the family Pseudomonadaceae (such as *Pseudomonas aeruginosa*)*,* Bacteroides species (such as *Bacteroides fragilis*)*,* Haemophilus species (such as *Haemophilus influenzae* and *Haemophilus parainfluenzae*)*,* Helicobacter species (such as *Helicobacter pylori*)*,* Neisseria species (such as *Neisseria gonorrhoeae* and *Neisseria meningitidis*), *Campylobacter jejuni,* Legionella species (such as *Legionella pneumophila, Moraxella catarrhalis,* Bacillus species (such as *Bacillus subtilis*), Enterococcus species (such as *Enterococcus faecalis* and *Enterococcus faecium*), Staphylococcus species (such as *Staphylococcus aureus and Staphylococcus epidermidis*)*,* Streptococcus species (such as *Streptococcus pneumoniae, Streptococcus mutans, and Streptococcus pyogenes*), *Mycoplasma pneumoniae,* and *Clostridium difficile.*

In some embodiments, the DNA binding subunit is from a Gram-negative prokaryote and the ATP-hydrolyzing subunit is from *S. aureus*

Gram-negative prokaryotes include the family Enterobacteriaceae (such as *E. coli, Klebsiella pneumoniae,* and *Salmonella typhimurium*), the family Pseudomonadaceae (such as *Pseudomonas aeruginosa*), Bacteroides species (such as *Bacteroides fragilis*), Haemophilus species (such as *Haemophilus influenzae* and *Haemophilus parainfluenzae*), Helicobacter species (such as *Helicobacter pylori),* Neisseria species (such as *Neisseria gonorrhoeae* and *Neisseria meningitidis*), *Campylobacter jejuni,* Legionella species (such as *Legionella pneumophila*), and *Moraxella catarrhalis.*

In some embodiments, the DNA binding subunit is *E. coli* GyrA and the ATP-hydrolyzing subunit is *S. aureus* GyrB.

In some embodiments, the DNA binding subunit is *E. coli* GyrA and the ATP-hydrolyzing subunit is *S. aureus* ParE.

In another aspect, the present invention provides methods of assaying the activity of topoisomerases.

In some embodiments, topoisomerase activity is assayed by combining a hybrid topoisomerase comprising a DNA binding subunit of a type II topoisomerase from one source (species or topoisomerase) and an ATP-hydrolyzing subunit of a type II topoisomerase from another source (species or topoisomerase) determining topoisomerase activity.

Topoisomerase activity can be determined using any method or assay. Many such methods are known to those of skill in the art. For example, topoisomerase activity can be determined by detecting ATPase activity; see for example *DNA Topoisomerase Protocols,* Osheroff & Bjomsti eds., Methods in Molecular Biology, Vol. 95 (2001). ATPase activity can be determined, detected, measured or quantified in many ways known to those of skill in the art, including, but not limited to, quantitating inorganic orthophosphate (phosphate) or adenosine diphosphate (ADP). Alternatively, topoisomerase activity can be determined by looking at changes in DNA topology (including DNA relaxation, supercoiling or decatenation).

In another aspect, the present invention provides methods for identifying compounds that modulate topoisomerase activity using hybrid topoisomerases.

As used herein, the terms "modulate" or "modulates" in reference to topoisomerase activity includes any measurable alteration, either an inhibition or enhancement, of topoisomerase activity.

In some embodiments, compounds are identified by providing a hybrid topoisomerase comprising a DNA binding subunit from a prokaryotic type II topoisomerase and an ATP-hydrolyzing subunit from a prokaryotic type II topoisomerase, contacting the hybrid topoisomerase with a test compound, and determining topoisomerase activity, wherein a change in topoisomerase activity in the presence of the compound as compared with topoisomerase activity in the absence of the compound indicates that the compound modulates topoisomerase activity. Topoisomerase modulators are identified as those compounds that, when present, result in an alteration in activity of the hybrid topoisomerase.

The present invention also provides methods for identifying antibacterial agents based upon the identification of compounds that inhibit topoisomerase activity or topoisomerase ATPase activity, using the hybrid toposiomerases of the present invention.

In some embodiments, compounds that are identified as capable of modulating topoisomerase activity are further tested for the ability to kill or inhibit the growth of bacteria. Assays of antibacterial activity are well known to those of skill in the art.

Topoisomerase and gyrase proteins can be obtained for use in the present invention according to procedures well known to the art. Topoisomerase and gyrase proteins can be obtained by isolation or purification from natural sources or expressed using recombinant technology. Numerous techniques for obtaining topoisomerase and gyrase proteins and subunite, including bacterial type II topoisomerase proteins and subunits, have been described in the literature (Staudenbauer & Orr, 1981, Nucleic Acids Res., 9:3589-3603; Brockbank & Barth, 1993, J. Bacteriol., 175:3269-3277; Gootz *et al.,* 1999, Antimicrob. Agents Chemother., 43:1845-1855; Mizuuchi *et al.,* 1984, J. Biol. Chem., 259:9199-9201; Hallett *et al.,* 1990, Gene, 93:139-142). These preparations also include methods where affintity tag-modified topoisomerases have been prepared by standard recombinant DNA methods to ease the purification of the resulting protein (Pan & Fisher, 1999, Antimicrob. Agents Chemother., 1999, 43:1129-36; Gross *et al.,* 2003, Antimicrob. Agents Chemother., 47:1037-46). Sequence information for identified topoisomerase subunit polypeptides and the genes and cDNAs encoding topoisomerase subunit polypeptides can be found in databases such as GenBank.

Generally, high purity protein preparations (>95%) are desired. However, less pure preparations can be used, provided they do not contain high levels of other ATP-hydrolyzing enzymes.

Enzymes that hydrolyze ATP to ADP and inorganic phosphate are common, and as a consequence, methods to detect the reaction products are well known in the literature. These methods can be used with the assays of the present invention for monitoring topoisomerase ATPase activity. Such methods include, but are not limited to, the ammonium molybdate / malachite green-based phosphate detection system (Lanzetta *et al.,* Anal. Biochem., 100:95-97; Cogan *et al.,* 1999 Anal. Biochem., 271:29-35), the methyl thio guanosine (MESG) / nucleoside phosphorylase reaction (Banik & Roy, 1990, Biochem. J., 266:611-614; Ali *et al.,* 1993, Biochemistry, 32:2717-2724), radioactive ³²P based detection (Sugino & Cozzarelli, 1980, J. Biol. Chem., 255:6299-6306) (including proximity-effect scintillation) (Jeffery *et al.,* 2002, Anal. Biochem., 304:55-62), and the pyruvate kinase / lactate dehydrogenase coupled enzyme system (Ali *et al.,* 1993, Biochemistry, 32:2717-2724; Tamura & Gellert, 1990, J. Biol. Chem., 265:21342-21349).

Purified *S. aureus* GyrB does not have a highly active ATPase. Blanche *et al.* (1996, Antimicrob. Agents Chemother., 40:2714-2720) have reported that the DNA supercoiling activity of *S. aureus* gyrase (containing both GyrA and GyrB proteins) can be increased ~500 fold by the addition of high concentrations (0.7 M) of potassium glutamate. Addition of this salt to an assay buffer containing *S. aureus* GyrB results in an increase in activity, on the order of about 10-fold. We find that addition of equimolar *E. coli* GyrA to the *S. aureus* GyrB protein in the absence of potassium glutamate (K glu) increases the ATPase activity about 20-fold. DNA further activates the cross-species GyrA/GyrB mixture for a total increase over the initial GyrB ATPase activity of about 620-fold.

The ATPase activity of *S. aureus* ParE is also enhanced by the addition of *E. coli* GyrA and DNA. DNA added to *S. aureus* ParE enhances the ATPase activity over 2-fold. Co-treatment of *S. aureus* ParE with DNA and *E. coli* GyrA activates the cross-species GyrA/ParE mixture for a total increase over the initial ParE activity of about 4.3-fold.

In some embodiments of the assays of the present invention, DNA is included with the protein subunits. A variety of different types of double stranded DNA can be used with the methods of the present invention, including, but not limited to, sonicated salmon sperm DNA, annealed synthetic oligonucleotides, or plasmid DNA being either nicked, or closed circular either supercoiled or relaxed. It will be appreciated by those of skill in the art that gyrase enzymes exhibit some sequence- and length-specificity with regard to their polynucleotide substrate (Fisher *et al.,* 1981, Proc. Natl. Acad. Sci. USA, 78:4165-419; Lockshon & Morris, 1985, J. Mol. Biol., 181:63-74; Lother *et al.,* 1984, Nucleic Acids Res., 12:901-914; Morrison *et al.,* 1980, J. Biol. Chem., 255:2211-2219). A variety of different types of DNA can be used with similar results including naturally occurring or synthetic, linear or closed-circular. For closed circular DNA, supercoiled or relaxed topologies are both acceptable as is nicked. The exact choice of DNA used may impact the optimal concentration of DNA as well as the magnitude of ATPase activation to a slight degree, but adjustments are well within the skill of the art.

In some embodiments, the DNA binding subunit and ATP hydrolyzing subunit combined in equimolar mixtures. In some embodiments, the DNA binding subunit and ATP hydrolyzing subunit combined in equimolar mixtures. For example, use of equimolar quantities of *E. coli* GyrA and *S. aureus* GyrB or ParE in the presence of DNA is a highly economical composition in terms of protein usage for the assay of the ATPase activity. Alternately, non-equimolar mixtures of the *E. coli* GyrA and *S. aureus* GyrB or ParE also result in ATPase activation and can be employed, although with a lower degree of efficiency.

As used herein, the phrases "conditions that promote topoisomerase activity," "conditions that promote topoisomerase ATPase activity" and "conditions that promote specific ATP hydrolyzing activity" refer to reaction conditions that support the catalytic activity of the topoisomerases of the present invention. Assays of the present invention are conducted under conditions such that hybrid topoisomerases have some or all of their catalytic activities. Such conditions are known to those of skill in the art. Mg²⁺ is important to support catalytic activity of type II topoisomerases, generally in a range of about 1 to about 20 mM, more particularly from about 3 to about 8 mM. Catalytic activity is supported by a broad pH range of about pH 6 to about pH 9, more particularly, from about pH 7 to about pH 8. In some embodiments of the present invention the pH ranges from about pH 7 to about pH 8. Suitable pH buffering agents include, but are not limited to, tris(hydroxyethyl)aminomethane (TRIS), N-(2-hydroxyethyl)piperazine-N'-(2-ethanesulfonic acid) (HEPES), and 3-(N-morpholino)propanesulfonic acid (MOPS) at a concentration of about 10 to about 100 mM.

In some embodiments of the present invention, a supporting electrolyte is included at concentrations ranging from about 25 mM to about 150 mM (examples of which include, but are not limited to, sodium chloride, potassium chloride, or ammonium chloride, sodium acetate, potassium acetate, or ammonium acetate). In some embodiments, about 50 mM to about 90 mM ammonium acetate is included.

In some embodiments of the present invention, metal scavenging reagents such as ethylenediaminetetraacetic acid (EDTA) or phenanthroline are optionally included. If such metal scavenging reagents are included, they should not be used in great excess over magnesium ion.

In some embodiments of the present invention, reducing agents such as tris(carboxyethylphosphine) (TCEP) or dithiothreitol (DTT) may also be included, ranging in concentration from about 1 mM to about 10 mM. The polyamine spermidine may be included (from about 2 mM to about 5 mM) as well as stabilizing agents such as glycerol (ranging from about 2 to about 10 % w/v) or bovine serum albumin.

The invention is further illustrated by way of the following examples, which are intended to elaborate several embodiments of the invention. These examples are not intended to, nor are they to be construed to, limit the scope of the invention. It will be clear that the invention may be practiced otherwise than as particularly described herein. Numerous modifications and variations of the present invention are possible in view of the teachings herein and, therefore, are within the scope of the invention.

### EXAMPLES

### Example 1. Methods.

Genes encoding the GyrA protein from *E. coli* and the GyrB protein from *S. aureus* were amplified from genomic DNA using the polymerase chain reaction with primers based on available sequence information from GenBank. The *E. coli gyrA* gene was cloned into an expression plasmid under the control of a *tac* promoter for recombinant expression in *E. coli.* The *S. aureus gyrB* gene was cloned into an expression plasmid under the control of a T7 promoter for recombinant expression in *E. coli.* Separate cultures of *E. coli* containing the expression plasmids were grown at 37°C to mid-log phase; isopropyl-beta-D-thiogalactopyranoside (IPTG) was added to induce expression for several hours. The recombinant *E. coli* GyrA and *S. aureus* GyrB were purified from bacterial cell lysates using a combination of ion exchange and size exclusion chromatography to yield >95% pure gyrase subunits.

Hybrid topoisomerase was formed by combining purified *E. coli* GyrA and *S. aureus* GyrB at a concentration of 1 µM each in a buffer containing 50 mM Tris (pH 7.5), 75 mM ammonium acetate, and 2 mg/mL sonicated salmon sperm DNA. The solution was gently mixed and was used immediately or stored at room temperature or on ice for several hours. The enzyme stock was then diluted 1:1000 in assay buffer that lacks ATP. The diluted enzyme stock was then dispensed at 50 µL per well into a standard, clear bottom, 96-well microplate. Dilution series of candidate ATPase modulators were prepared in DMSO, and 2 µL of these were dispensed into the enzyme-containing buffer. The enzyme reaction was initiated by the addition of 50 µL of 500 µM ATP in assay buffer (see table below), and allowed to proceed for 3 hours. At the end of that time, 150 µL per well of quench reagent was added and the solution was incubated for about 5 minutes. The extent of reaction was monitored by measuring the absorbance at 650 nm (A650). Along with the test compounds, wells that contained no compound and a positive control (2 µM novobiocin) were included to reference 0 and 100% inhibition. A "no reaction" assay plate was prepared in tandem with the assay plate containing all reaction components and the test compound but not the enzyme. The enzyme-induced change in A650 between the two test plates was then analyzed for inhibition of the *S. aureus* GyrB ATPase reaction.

### Assay conditions:

- Buffer:: 50 mM Trishydroxyethyl amino methane (TRIS) pH 7.5, 75 mM ammonium acetate, 5% w/v glycerol, 0.5mM EDTA, 5.5 mM magnesium chloride, 1 mM dithiothreitol, 200 nM (13 µg/mL) bovine serum albumin
- DNA:: 2 µg/mL (~3 µM base pairs)
- Enzyme:: 1 nM each *E. coli* GyrA, *S. aureus* GyrB
- Test compound:: 1:50 dilution from DMSO stock (2% final DMSO)
- ATP:: 250 µM
- Quench reagent:: 0.92 mM malachite green hydrochloride, 8.5 mM ammonium molybdate tetrahydrate in 1 N hydrochloric acid
- Readout:: Absorbance at 650 nm

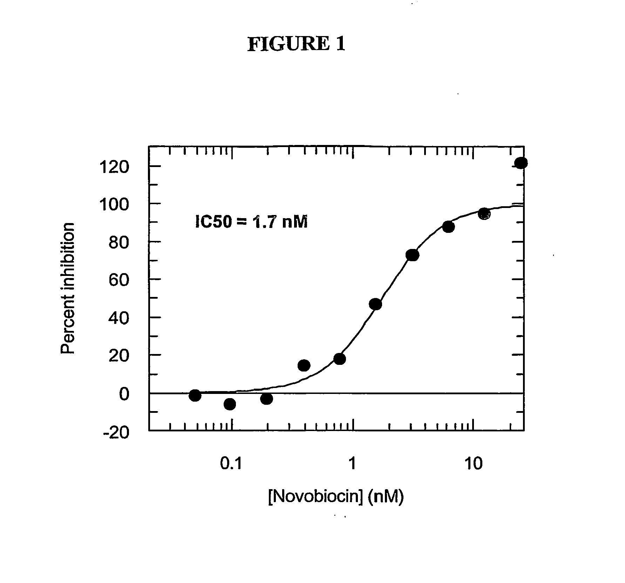

The results are shown in Figure 1. The described assay was performed upon the potent natural-product gyrase inhibitor, novobiocin, and an IC₅₀ curve generated. The IC₅₀ value obtained of 1.7 nM is in good agreement with the value of 2.2 nM for the dissociation constant of the novobiocin / hybrid gyrase complex determined separately. In addition, we have demonstrated with a set of diverse compounds, that there is a direct correlation between IC₅₀ values of ATPase inhibition using the hybrid enzyme assay, and antibacterial activity directed against *S. aureus.*

### Example 2. Assay of S. aureus GyrB activity in the presence of various effectors.

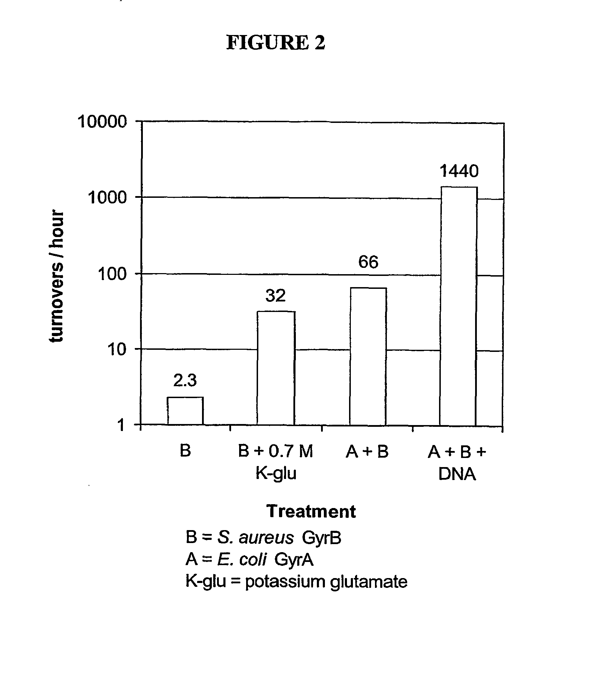

Illustration of the improvement in *S. aureus* GyrB ATPase activity via the reconstitution of the ATPase activity of *S. aureus GyrB* with *E. coli GyrA* is provided in Figure 2. Comparisons of the ATPase activity of different treatments of *S. aureus* GyrB were made in similar buffers, and total enzyme concentrations were varied such that an easily measurable quantity of phosphate (~2-20µM) was produced in an hour or less. All measurements contained 50 mM TRIS at pH 7.5, 5% glycerol, 75-100 mM electrolyte (either sodium chloride or ammonium acetate), 0.5mM EDTA, 5.5 mM magnesium chloride, 1 mM DTT, and 300 µM ATP. Concentrations of *S. aureus* GyrB varied from 600 nM to 12.5 nM in these comparisons. When *E. coli* GyrA was added, it was present at the same molar concentration as the *S. aureus* GyrB. When present, K glu was at a concentration of 0.7 M. For measurements containing DNA, 25 µg/mL sonicated salmon sperm DNA was present.

### Example 3. Assay of the effect of DNA on the potency of E. coli GyrA as an activator of S. aureus GyrB.

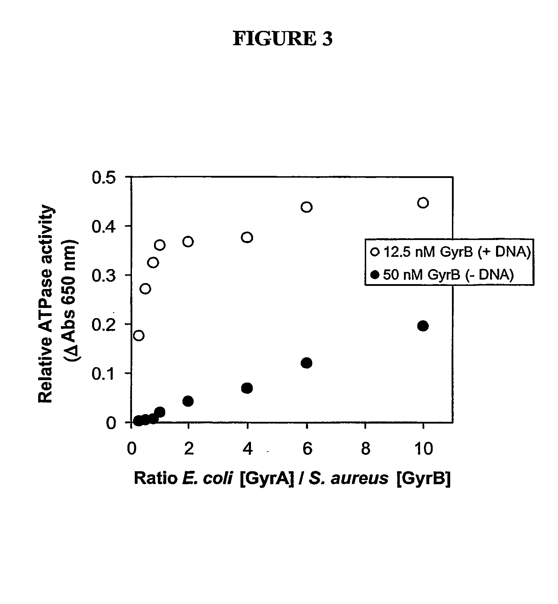

The effect of varying amounts of *E. coli* GyrA relative to *S. aureus* GyrB is further exemplified in Figure 3. The activities of mixtures of various ratios of the two proteins were measured using the methods essentially as described above in Example 1. In the absence of DNA, excesses of *E. coli* GyrA are required to substantially increase the activity of *S. aureus* GyrB. In contrast, when DNA is present the stimulatory effect of *E. coli* GyrA is potentiated, and is substantially complete with a single equivalent of the activating protein.

### Example 4. Assay of plasmid DNA supercoiling with S. aureus GyrB and E. coli GyrA.

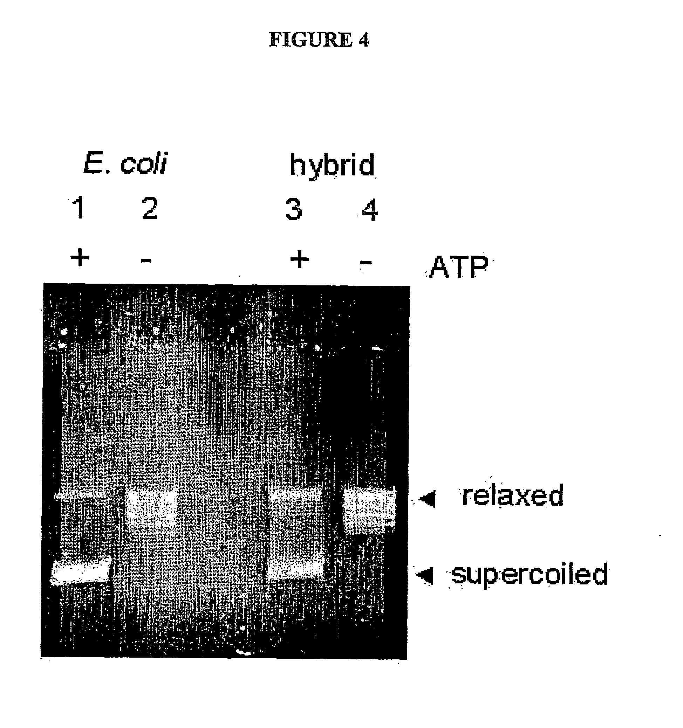

Illustration of the ATP dependence of supercoiling of the *E. coli* GyrA and *S. aureus* GyrB hybrid is provided in Figure 4. Lanes 1 and 2 show *E. coli* gyrase (*E.coli* GyrA / *E. coli* GyrB) supercoiling activity in the presence and absence of ATP respectively. Lanes 3 and 4 show the analogous results for the *E. coli* GyrA / *S. aureus* GyrB cross-species hybrid. The observation of ATP-dependent supercoiling of relaxed plasmid DNA established that the cross-species hybrid performed the complete reaction of DNA gyrase.

Supercoiling reactions of *E. coli* gyrase and the *E. coli* GyrA and *S. aureus* GyrB hybrid were conducted with 50 µL samples containing 0.5 µg of relaxed pBR322 plasmid DNA and 20 nM each of the appropriate GyrA and GyrB subunits. Reactions, performed at room temperature, were initiated by the addition of ATP to a final concentration of 1 mM and allowed to proceed for 20 minutes. Reactions were quenched with agarose gel-loading buffer (10 µL), and 20 µL of this solution analyzed by gel electrophoresis and ethidium bromide fluorescence under ultraviolet transillumination. Buffer composition is described below.

### Assay conditions:

- Buffer:: 50 mM 4-(2-Hydroxyethyl)piperazine-1-ethanesulfonic acid (HEPES) pH 7.5, 75 mM ammonium acetate, 5% w/v glycerol, 0.5mM EDTA, 5.5 mM magnesium chloride, 1 mM dithiothreitol, 200 nM (13 µg/mL) bovine serum albumin, 2 mM spermidine
- DNA:: 0.5 µg relaxed pBR322
- Enzyme:: 20 nM each *E. coli* GyrA; *E. coli* or *S. aureus* GyrB as appropriate
- ATP:: 1 mM, when present
- Quench reagent:: 30% w/v Ficoll 400 (Pharmacia), 100 mM EDTA, 5% w/v sodium dodecyl sulfate, 0.2 µg/mL bromphenol blue
- Readout:: Agarose gel electrophoresis (0.8% w/v) run at 3 V/cm for 3 hours in a running buffer of 90 mM TRIS, 90 mM boric acid, 2 mM EDTA (1x TBE). After electrophoresis, the DNA was stained in 5 µg/mL ethidium bromide and photographed under ultraviolet transillumination.

### Example 5. Assay of S. aureus ParE activity in the presence of various effectors.

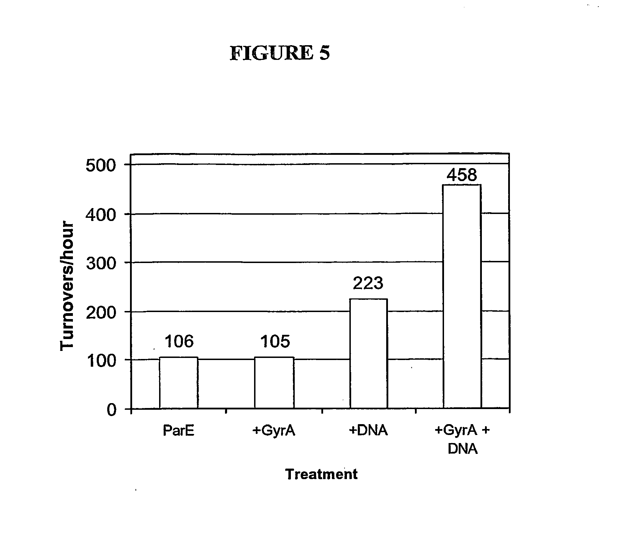

Illustration of the improvement in *S. aureus* ParE ATPase activity via the reconstitution of *S. aureus* ParE with *E. coli GyrA* and DNA is provided in Figure 5. Comparisons of the ATPase activity of different treatments of *S. aureus* ParE were made in the following buffer; 50 mM TRIS pH 7.5, 75 mM ammonium acetate, 5% w/v glycerol, 0.5mM EDTA, 5.5 mM magnesium chloride, 1 mM dithiothreitol, 200 nM (13 µg/mL) bovine serum albumin. The total concentration of each enzyme subunit (as appropriate) was 100 nM. For the measurement containing DNA, 200 µg/mL sonicated salmon sperm DNA was present. Reactions were initiated by the addition of ATP to a final concentration of 500 µM and allowed to proceed for 30 minutes after which time they were quenched and analyzed for phosphate production as described in Example 1. The ATPase activity of the *S. aureus* ParE was enhanced by DNA. In the presence of equimolar *E. coli* GyrA, this enhancement was potentiated.

## Claims

1. A hybrid topoisomerase comprising a DNA binding subunit of a type II topoisomerase from a Gram-negative prokaryote and an ATP-hydrolyzing subunit of a type II topoisomerase from *S. aureus.*

2. The hybrid topoisomerase of claim 1, wherein the Gram-negative prokaryote is selected from Enterobacteriaceae, Pseudomonadaceae, Bacteroides species, Haemophilus species, Helicobacter species, Neisseria species, *Campylobacter jejuni,* Legionella species, and *Moraxella catarrhalis.*

3. The hybrid topoisomerase of claim 1, wherein the Gram-negative prokaryote is *E. coli.*

4. The hybrid topoisomerase of claim 1, wherein the type II topoisomerase DNA binding subunit is GyrA from *E. coli.*

5. The hybrid topoisomerase of claim 1, wherein the ATP-hydrolyzing subunit is *S. aureus* GyrB or ParE.

6. The hybrid topoisomerase of claim 1, wherein the hybrid topoisomerase comprises *E. coli* GyrA and *S. aureus* GyrB.

7. The hybrid topoisomerase of claim 1, wherein the hybrid topoisomerase comprises *E. coli* GyrA and *S. aureus* ParE.

8. A method for assaying topoisomerase activity comprising:
a) providing the hybrid topoisomerase of claim 1; and
b) determining topoisomerase activity.

9. The method of claim 8, further comprising contacting the hybrid topoisomerase with DNA after step a).

10. The method of claim 9, wherein topoisomerase activity is determined by detecting a change in topology of the DNA.

11. The method of claim 10, wherein the change in DNA topology is determined by detecting DNA relaxation, DNA supercoiling, or DNA decatenation.

12. The method of claim 8, wherein topoisomerase activity is determined by detecting ATPase activity.

13. The method of claim 12, wherein ATPase activity is detected by measuring inorganic orthophosphate or adenosine diphosphate.

14. The method of claim 8, wherein the hybrid topoisomerase comprises *E. coli* GyrA and *S. aureus* GyrB.

15. The method of claim 8, wherein the hybrid topoisomerase comprises *E. coli* GyrA and *S. aureus* ParE.

16. A method for identifying compounds that modulate topoisomerase activity comprising:
a) providing a hybrid topoisomerase comprising a DNA binding subunit from a prokaryotic type II topoisomerase and an ATP-hydrolyzing subunit from a prokaryotic type II topoisomerase;
b) contacting the hybrid topoisomerase with a test compound; and
c) determining topoisomerase activity, wherein a change in topoisomerase activity in the presence of said compound as compared with topoisomerase activity in the absence of said compound indicates that said compound modulates topoisomerase activity.

17. The method of claim 16, further comprising contacting the hybrid topoisomerase with DNA and a test compound.

18. The method of claim 17, wherein topoisomerase activity is determined by detecting a change in topology of the DNA.

19. The method of claim 18, wherein the change in DNA topology is determined by detecting DNA relaxation, DNA supercoiling, or DNA decatenation.

20. The method of claim 16, wherein topoisomerase activity is determined by detecting ATPase activity.

21. The method of claim 20, wherein ATPase activity is detected by measuring inorganic orthophosphate or adenosine diphosphate.

22. The method of claim 16, further comprising determining if the compound has antibacterial activity.

23. The method of claim 16, wherein the DNA binding subunit is *E. coli* GyrA and the ATP-hydrolyzing subunit is *S. aureus* GyrB.

24. The method of claim 16, wherein the DNA binding subunit is *E. coli* GyrA and the ATP-hydrolyzing subunit is *S. aureus* ParE.

## Patentansprüche

1. Hybrid-Topoisomerase, umfassend eine DNA bindende Untereinheit einer Typ-II-Topoisomerase aus einem gramnegativen Prokaryonten sowie eine ATP hydrolysierende Untereinheit einer Typ-II-Topoisomerase aus *S. aureus.*

2. Hybrid-Topoisomerase nach Anspruch 1, wobei der gramnegative Prokaryont ausgewählt ist aus Enterobacteriaceae, Pseudomonadaceae, Bacterioides-Spezies, Haemophilus-Spezies, Helicobacter-Spezies, Neisseria-Spezies, *Campylobacter jejuni,* Legionella-Spezies und *Moraxella catarrhalis.*

3. Hybrid-Topoisomerase nach Anspruch 1, wobei es sich bei dem gramnegativen Prokaryonten um *E. coli* handelt.

4. Hybrid-Topoisomerase nach Anspruch 1, wobei es sich bei der DNA bindenden Untereinheit der Typ-II-Topoisomerase um GyrA aus *E. coli* handelt.

5. Hybrid-Topoisomerase nach Anspruch 1, wobei es sich bei der ATP hydrolysierenden Untereinheit um GyrB oder ParE aus *S. aureus* handelt.

6. Hybrid-Topoisomerase nach Anspruch 1, wobei diese GyrA aus *E. coli* und GyrB aus *S. aureus* umfaßt.

7. Hybrid-Topoisomerase nach Anspruch 1, wobei diese GyrA aus *E. coli* und ParE aus *S. aureus* umfaßt.

8. Verfahren zum Testen von Topoisomeraseaktivität, bei dem man:
a. die Hybrid-Topoisomerase nach Anspruch 1 bereitstellt und
b. Topoisomeraseaktivität bestimmt.

9. Verfahren nach Anspruch 8, bei dem man ferner nach dem Schritt a) die Hybrid-Topoisomerase mit DNA in Kontakt bringt.

10. Verfahren nach Anspruch 9, wobei Topoisomeraseaktivität bestimmt wird, indem man eine Änderung der Topologie der DNA nachweist.

11. Verfahren nach Anspruch 10, wobei die Änderung der DNA-Topologie durch Nachweisen von DNA-Relaxation, DNA-Supercoiling oder DNA-Decatenierung bestimmt wird.

12. Verfahren nach Anspruch 8, wobei Topoisomeraseaktivität bestimmt wird, indem man ATPase-Aktivität nachweist.

13. Verfahren nach Anspruch 12, wobei man ATPase-Aktivität durch Messen von anorganischem Orthophosphat oder Adenosindiphosphat nachweist.

14. Verfahren nach Anspruch 8, wobei die Hybrid-Topoisomerase GyrA aus *E. coli* und GyrB aus *S. aureus* umfaßt.

15. Verfahren nach Anspruch 8, wobei die Hybrid-Topoisomerase GyrA aus *E. coli* und ParE aus *S. aureus* umfaßt.

16. Verfahren zur Identifizierung von Topoisomeraseaktivität modulierenden Verbindungen, bei dem man:
a. eine Hybrid-Topoisomerase, umfassend eine DNA bindende Untereinheit aus einer prokaryontischen Typ-II-Topoisomerase sowie eine ATP hydrolysierende Untereinheit aus einer prokaryontischen Typ-II-Topoisomerase bereitstellt,
b. die Hybrid-Topoisomerase mit einer Testverbindung in Kontakt bringt und
c. Topoisomeraseaktivität bestimmt, wobei eine Änderung der Topoisomeraseaktivität in Gegenwart der Verbindung gegenüber der Topoisomeraseaktivität in Abwesenheit der Verbindung darauf hindeutet, daß die Verbindung Topoisomeraseaktivität moduliert.

17. Verfahren nach Anspruch 16, bei dem man ferner die Hybrid-Topoisomerase mit DNA sowie einer Testverbindung in Kontakt bringt.

18. Verfahren nach Anspruch 17, wobei Topoisomeraseaktivität bestimmt wird, indem man eine Änderung der Topologie der DNA nachweist.

19. Verfahren nach Anspruch 18, wobei die Änderung der DNA-Topologie durch Nachweisen von DNA-Relaxation, DNA-Supercoiling oder DNA-Decatenierung bestimmt wird.

20. Verfahren nach Anspruch 16, wobei Topoisomeraseaktivität bestimmt wird, indem man ATPase-Aktivität nachweist.

21. Verfahren nach Anspruch 20, wobei man ATPase-Aktivität durch Messen von anorganischem Orthophosphat oder Adenosindiphosphat nachweist.

22. Verfahren nach Anspruch 16, bei dem man ferner bestimmt, ob die Verbindung antibakterielle Aktivität aufweist.

23. Verfahren nach Anspruch 16, wobei es sich bei der DNA bindenden Untereinheit um GyrA aus *E. coli* und bei der ATP hydrolysierenden Untereinheit um GyrB aus *S. aureus* handelt.

24. Verfahren nach Anspruch 16, wobei es sich bei der DNA bindenden Untereinheit um GyrA aus *E. coli* und bei der ATP hydrolysierenden Untereinheit um ParE aus *S. aureus* handelt.

## Revendications

1. Topoisomérase hybride comprenant une sous-unité de liaison à l'ADN d'une topoisomérase de type II provenant d'un procaryote Gram-négatif et une sous-unité d'hydrolyse de l'ATP d'une topoisomérase de type II provenant de *S. aureus.*

2. Topoisomérase hybride selon la revendication 1, **caractérisée en ce que** le procaryote Gram-négatif est choisi parmi les Enterobacteriaceae, les Pseudomonadaceae, les espèces Bacteroides, les espèces Haemophilus, les espèces Helicobacter, les espèces Neisseria, *Campylobacter jejuni,* les espèces Legionella et *Moraxella catarrhalis.*

3. Topoisomérase hybride selon la revendication 1, **caractérisée en ce que** le procaryote Gram-négatif est *E. coli.*

4. Topoisomérase hybride selon la revendication 1, **caractérisée en ce que** la sous-unité de liaison à l'ADN de topoisomérase de type II est GyrA d'*E. coli.*

5. Topoisomérase hybride selon la revendication 1, **caractérisée en ce que** la sous-unité d'hydrolyse de l'ATP est GyrB ou ParE de *S. aureus.*

6. Topoisomérase hybride selon la revendication 1, **caractérisée en ce que** la topoisomérase hybride comprend GyrA d'*E*. *coli* et GyrB de *S. aureus.*

7. Topoisomérase hybride selon la revendication 1, **caractérisée en ce que** la topoisomérase hybride comprend GyrA d'*E. coli* et ParE de *S. aureus.*

8. Procédé de dosage de l'activité topoisomérase, comprenant les étapes consistant à:
a) fournir la topoisomérase hybride selon la revendication 1; et
b) déterminer l'activité topoisomérase.

9. Procédé selon la revendication 8, comprenant en outre l'étape consistant à mettre la topoisomérase hybride en contact avec de l'ADN après l'étape a).

10. Procédé selon la revendication 9, **caractérisé en ce que** l'activité topoisomérase est déterminée par détection d'un changement de topologie de l'ADN.

11. Procédé selon la revendication 10, **caractérisé en ce que** le changement de topologie de l'ADN est déterminé par détection d'un relâchement de l'ADN, d'un superenroulement de l'ADN, ou d'une décaténation de l'ADN.

12. Procédé selon la revendication 8, **caractérisé en ce que** l'activité topoisomérase est déterminée par détection d'une activité ATPase.

13. Procédé selon la revendication 12, **caractérisé en ce que** l'activité ATPase est détectée par mesure de l'orthophosphate minéral ou de l'adénosine diphosphate.

14. Procédé selon la revendication 8, **caractérisé en ce que** la topoisomérase hybride comprend GyrA d'*E. coli* et GyrB de *S. aureus.*

15. Procédé selon la revendication 8, **caractérisé en ce que** la topoisomérase hybride comprend GyrA d'*E. coli* et ParE de *S. aureus.*

16. Procédé d'identification de composés qui modulent l'activité topoisomérase, comprenant les étapes consistant à:
a) fournir une topoisomérase hybride comprenant une sous-unité de liaison à l'ADN provenant d'une topoisomérase de type II procaryote et une sous-unité d'hydrolyse de l'ATP provenant d'une topoisomérase de type II procaryote;
b) mettre la topoisomérase hybride en contact avec un composé à tester; et
c) déterminer l'activité topoisomérase, un changement de l'activité topoisomérase en présence dudit composé par rapport à l'activité topoisomérase en l'absence dudit composé indiquant que ledit composé module l'activité topoisomérase.

17. Procédé selon la revendication 16, comprenant en outre l'étape consistant à mettre la topoisomérase hybride en contact avec de l'ADN et un composé à tester.

18. Procédé selon la revendication 17, **caractérisé en ce que** l'activité topoisomérase est déterminée par détection d'un changement de topologie de l'ADN.

19. Procédé selon la revendication 18, **caractérisé en ce que** le changement de topologie de l'ADN est déterminé par détection d'un relâchement de l'ADN, d'un superenroulement de l'ADN ou d'une décaténation de l'ADN.

20. Procédé selon la revendication 16, **caractérisé en ce que** l'activité topoisomérase est déterminée par détection d'une activité ATPase.

21. Procédé selon la revendication 20, **caractérisé en ce que** l'activité ATPase est détectée par mesure de l'orthophosphate minéral ou de l'adénosine diphosphate.

22. Procédé selon la revendication 16, comprenant en outre l'étape consistant à déterminer si le composé a une activité antibactérienne.

23. Procédé selon la revendication 16, **caractérisé en ce que** la sous-unité de liaison à l'ADN est GyrA d'*E*. *coli* et **en ce que** la sous-unité d'hydrolyse de l'ATP est GyrB de *S. aureus.*

24. Procédé selon la revendication 16, **caractérisé en ce que** la sous-unité de liaison à l'ADN est GyrA d'*E. coli* et **en ce que** la sous-unité d'hydrolyse de l'ATP est ParE de *S. aureus.*
